# EUROPEAN PATENT SPECIFICATION

(11) **EP 2 849 763 B1**
(45) Date of publication and mention of the grant of the patent: **25.10.2017**
(21) Application number: 13729822.0
(22) Date of filing: 16.05.2013
(51) Int. Cl.: A61K 31/722, A61P 31/14

(54) **THE USE OF CHITOSAN POLYMER IN THE TREATMENT AND PREVENTION OF INFECTIONS CAUSED BY CORONAVIRUSES**
DIE VERWENDUNG VON CHITOSANPOLYMEREN BEI DER BEHANDLUNG UND VORBEUGUNG VON CORONAVIRUS-ERKRANKUNGEN
POLYMERES DE CHITOSAN POUR LE TRAITEMENT ET LA PREVENTION DES INFECTIONS PROVOQUÉES PAR UNE ESPÈCE DU GENRE CORONA

(30) Priority: 18.05.2012 PL 39924612
(43) Date of publication of application: 25.03.2015
(73) Proprietor: Uniwersytet Jagiellonski, 31-007 Krakow (PL)
(72) Inventor: PYRC , Krzysztof, PL-31-231 Kraków (PL); MILEWSKA, Aleksandra, PL-31-979 Kraków (PL); NOWAKOWSKA, Maria, PL-30-433 Kraków (PL); SZCZUBIALKA, Krzysztof, PL-32-442 Krzywaczka (PL); KAMINSKI , Kamil, PL-34-220 Makow Podhalanski (PL)
(74) Representative: BRANDPAT Patent and Trademark Attorneys Chlebicka Czyz Galazkiewicz Ziolkowski Professional Partnership
(86) International application number: PCT/PL2013/000064
(87) International publication number: WO 2013/172725

(56) References cited:
- WO-A2-2005/074947
- WO-A2-2008/105934
- LOUBAKI E ET AL: "Chemical modification of chitosan by glycidyl trimethylammonium chloride. characterization of modified chitosan by <13>C- and <1>H-NMR spectroscopy", EUROPEAN POLYMER JOURNAL, PERGAMON PRESS LTD. OXFORD, GB, vol. 27, no. 3, 1 January 1991 (1991-01-01), pages 311-317, XP024052962, ISSN: 0014-3057, DOI: 10.1016/0014-3057(91)90111-Z [retrieved on 1991-01-01]
- LIM S-H ET AL: "Synthesis and antimicrobial activity of a water-soluble chitosan derivative with a fiber-reactive group", CARBOHYDRATE RESEARCH, PERGAMON, GB, vol. 339, no. 2, 22 January 2004 (2004-01-22), pages 313-319, XP004776763, ISSN: 0008-6215, DOI: 10.1016/J.CARRES.2003.10.024
- YOUBIN WU ET AL: "Thermal-sensitive hydrogel as adjuvant-free vaccine delivery system for H5N1 intranasal immunization", BIOMATERIALS, ELSEVIER SCIENCE PUBLISHERS BV., BARKING, GB, vol. 33, no. 7, 25 November 2011 (2011-11-25), pages 2351-2360, XP028442485, ISSN: 0142-9612, DOI: 10.1016/J.BIOMATERIALS.2011.11.068 [retrieved on 2011-11-30]
- ALEKSANDRA MILEWSKA ET AL: "Novel polymeric inhibitors of HCoV-NL63", ANTIVIRAL RESEARCH, vol. 97, no. 2, 1 February 2013 (2013-02-01), pages 112-121, XP055084080, ISSN: 0166-3542, DOI: 10.1016/j.antiviral.2012.11.006

### SEQUENCE LISTING

<110> Jagiellonian University
<120> The use of chitosan polymer in the treatment and prevention of infections caused by coronaviruses.
<130> 20130516/UJ/PCT/4
<140> PCT/PL2013/000064
   <141> 2013-05-16
<150> PL P.399246
   <151> 2012-05-18
<160> 3
<170> PatentIn version 3.5
<210> 1
   <211> 24
   <212> DNA
   <213> Artificial Sequence
<220>
   <223> Mouse Hepatitis Virus (synthetic DNA forward primer)
<300>
   <301> Milewska A, Ciejka J, Kaminski K, Karewicz A, Bielska D, Zeglen S, Karolak W, Nowakowska M, Potempa J, Bosch BJ, Pyrc K, Szczubialka K. <302> Novel polymeric inhibitors of HCoV-NL63. <303> Antiviral Research <304> 97 <305> 2 <306> 112-121 <307> 2012-11-29
<400> 1
   tggccgaaga aattgctgct cttg 24
<210> 2
   <211> 27
   <212> DNA
   <213> Artificial Sequence
<220>
   <223> Mouse Hepatitis Virus (synthetic DNA reverse primer)
<300>
   <301> Milewska A, Ciejka J, Kaminski K, Karewicz A, Bielska D, Zeglen S, Karolak W, Nowakowska M, Potempa J, Bosch BJ, Pyrc K, Szczubialka K. <302> Novel polymeric inhibitors of HCoV-NL63. <303> Antiviral Research <304> 97 <305> 2 <306> 112-121 <307> 2012-11-29
<400> 2
   gcctgacttc tttggtggca ctttgct 27
<210> 3
   <211> 26
   <212> DNA
   <213> Artificial Sequence
<220>
   <223> Mouse Hepatitis Virus (synthetic DNA probe labelled with FAM and TAMRA dyes)
<300>
   <301> Milewska A, Ciejka J, Kaminski K, Karewicz A, Bielska D, Zeglen S, Karolak W, Nowakowska M, Potempa J, Bosch BJ, Pyrc K, Szczubialka K. <302> Novel polymeric inhibitors of HCoV-NL63. <303> Antiviral Research <304> 97 <305> 2 <306> 112-121 <307> 2012-11-29
<400> 3
   tttggctaag ctcggtaaag atgccg 26

## Description

The present invention is related to the chitosan polymers for use in the treatment of infections caused by coronaviruses by inhibiting their replication. Human viruses belonging to the *Coronaviridae* family cause respiratory infections manifested by insufficiency and impairment of correct respiratory functions. Some coronaviruses may also infect many animal species (including bats, mice, rats, cats, dogs, rabbits, horses, cattle, chickens, turkeys and wild birds), causing a wide range of diseases, affecting, among others, respiratory system, digestive tract, liver, cardiovascular system and nervous system.

In particular, the invention includes the chitosan polymers for use in the treatment of upper respiratory tract infections, infections of the lower respiratory tract, croup, gastrointestinal infections, infections of the nervous system, liver infections, infections of the cardiovascular system and Kawasaki disease in humans and animals. Coronaviruses are enveloped viruses having a genome in the form of a positive-sense single stranded RNA. Initially, the *Coronaviridae* family was divided into three groups based on serological methods.

Following implementation of molecular techniques this virus family was re-organized and at present it consists of alpha-, beta-, gamma- and deltacoronaviruses.

Alphacoronaviruses include numerous animal pathogens (e.g., feline peritonitis virus, porcine epidemic diarrhea virus) and two human coronaviruses (HCoV-229E and HCoV -NL63). The betacoronaviruses include animal viruses, such as mouse hepatitis virus (MHV), equine coronavirus, canine coronavirus, and three human coronaviruses (HCoV -OC43, HCoV-HKU1, and SARS-CoV). The gamma- and delta- coronaviruses include pathogens infecting birds and marine mammals.

The prior art knows the ability of chitosan and its derivatives to bind viruses and inhibit their replication. For example, it is known that sugar derivatives of chitosan possess the ability to bind the influenza virus via haemagglutinin protein, which is typical protein of influenza virus capable of binding sugar residues (Li X. et al. (2011). Biomacromolecules, 12: 3962 - 3969; Kalashnikova I. et al. (2008). Anal Chem 80: 2188 - 2198, Makimura Y et al. (2006). Carbohydr Res 341: 1803 - 1808, U.S. 6,680,054].

Similarly, unmodified chitosan in the form of microgranules was used to inhibit replication of foot-and-mouth disease (FMDV) infection. It has been shown that the administration of chitosan in the form of microgranules to suckling mice resulted in the reduction of disease symptoms (Li D. (2010). Virol J 7: 125).

It was also shown that sulfonated chitosan derivatives have antiviral activity inhibiting the replication of HIV-1 virus, probably by blocking the interaction between the gp120 viral protein and its receptor located on the surface of susceptible cells (CD4) (Artan M. et al. (2010). Carbohydr Res 345: 656 - 662). There are literature reports that sulfonic acid derivatives of chitosan inhibit replication of other retroviruses and members of the *Herpesviridae* family (U.S. 2009286757, U.S. 2005209189, EP 0497988, DE 4242813 and C. Ishihara et al. (1993). Vaccine 11: 670 - 674). Similarly, low molecular weight chitosan has antiviral activity against feline calicivirus and murine norovirus (Su X. et al. (2009). J Food Prot 72: 2623 - 2628). There are also patent applications and patents related to the antiviral activity of chitosan and chitosan derivatives.

However, soluble chitosan as well as sulfonic acid derivatives of chitosan, show no activity against coronaviruses. Similarly, the use of other cationically modified polymers did not result in inhibiting viral replication in in vitro conditions. The developed polymer, in contrast to those described above, showed no activity against other human viruses such as human metapneumovirus, enterovirus, influenza viruses A and B, and human herpesvirus type 1.

WO2005/074947 A2 discloses an antiviral efficiency of composition consisting of an ionic multivalent metal component, a cationic polymer, and a cationic surfactant. However, the cited invention does not provide any information about the activity of quaternary amine chitosan in the treatment and prevention of infections caused by the coronaviruses.

WO2008/105934 A2 refers to the use of chitosan and derivatives in inactivating human coronaviruses. Described application clearly indicates that the derivatives of chitosan are functionalized with amino acid, such as chitosan-arginine or chitosan-guanidine. Therefore, the mentioned invention differs from the subject-matter of the present application in the chitosan compound.

Publication LOUBAKI E ET AL: "Chemical modification of chitosan by glycidyl trimethylammonium chloride. characterization of modified chitosan by <13>C- and <1>H-NMR spectroscopy", EUROPEAN POLYMER JOURNAL, PERGAMON PRESS LTD. OXFORD, GB, vol. 27, no. 3, 1 (1991-01-01) discloses a method of chemical modification of chitosan by glycidyl trimethylammonium chloride whereas publication LIM S-H ET AL: "Synthesis and antimicrobial activity of a water-soluble chitosan derivative with a fiber-reactive group", CARBOHYDRATE RESEARCH, PERGAMON, GB, vol. 339, no. 2, 22 of January 2004 describes an antimicrobial activity of the chitosan derivative against a variety of bacteria and fungi. Both of the mentioned publications do not provide any information about antiviral activity of the modified chitosan in general, nor against the coronavirus in particular.

YOUBIN WU ET AL: "Thermal-sensitive hydrogel as adjuvant-free vaccine delivery system for H5N1 intranasal immunization", BIOMATERIALS, ELSEVIER SCIENCE PUBLISHERS BV., BARKING, GB, vol. 33, no. 7, 25 of November 2011 concerns use of chitosan derivatives as a carriers for vaccines against influenza virus H5N1. The chitosan derivatives due to its biocompatibility and mucoadhesive nature are used as a hydrogel for antigen deposition in nasal delivery. Disclosed work is not related to the antiviral properties of chitosan. Additionally, this publication does not mention the coronaviruses or the prevention or treatment of any such related diseases at all.

The aim of the invention is providing chitosan polymers for use in the treatment and prevention of infections caused by coronaviruses through inhibiting their replication. The essence of the invention is chitosan polymer of formula I, wherein R is selected from H or COCH₃ (HTCC) or a chitosan polymer of formula I, .
wherein R is selected from H, COCH₃, or C₁₂H₂₅ (HM-HTCC) for use in the treatment and prevention of infections caused by coronaviruses.

Preferably, the polymers are for use in the treatment and prevention of infections caused by alphacoronaviruses, and, particularly preferably, in the case of infections caused by HCoV-NL63.

Preferably, the polymers are for use in the treatment and prevention of infections caused by betacoronaviruses, and, particularly preferably, in the case of infections caused by murine coronavirus MHV.

Also preferable the polymers according to the invention are for use in the treatment and prevention of respiratory infections manifested by insufficiency and impairment of respiratory functions in humans or animals, particularly preferably in the treatment and prevention of infections of: upper respiratory tract infections, lower respiratory tract infections, croup in children, gastrointestinal infections, infections of the nervous system, and Kawasaki disease.

The chitosan polymer HTCC is obtained by reacting chitosan with glycidyltrimethylammonium chloride. HTCC, modified further by substituting with hydrophobic groups, particularly preferably when the hydrophobic groups are n-dodecyl groups, gives HM-HTCC.

These polymers are preferably used as solution in the form of a liquid or an aerosol administered topically to the upper respiratory tract (throat, nose, respiratory tree), orally to the gastrointestinal system, or intravenously in the treat and prevention of diseases caused by coronaviruses.

The subject of the invention are also the chitosan polymer of formula I, wherein R is H, or COCH₃ (HTCC) and the chitosan polymer of formula I, wherein R is H, COCH₃ or C₁₂H₂₅ (HM-HTCC) for use as solution in the form of a liquid or an aerosol administered to the respiratory tract.

The invention is illustrated in more detail by the application examples

### Example 1. Synthesis of the chitosan derivative HTCC

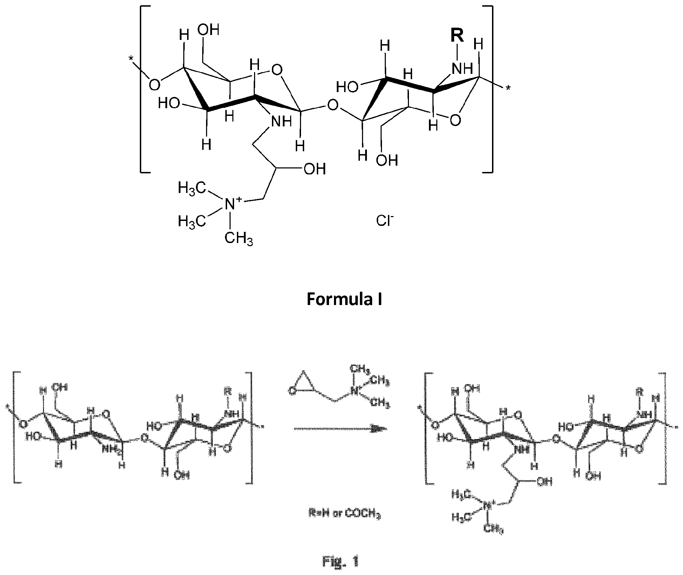

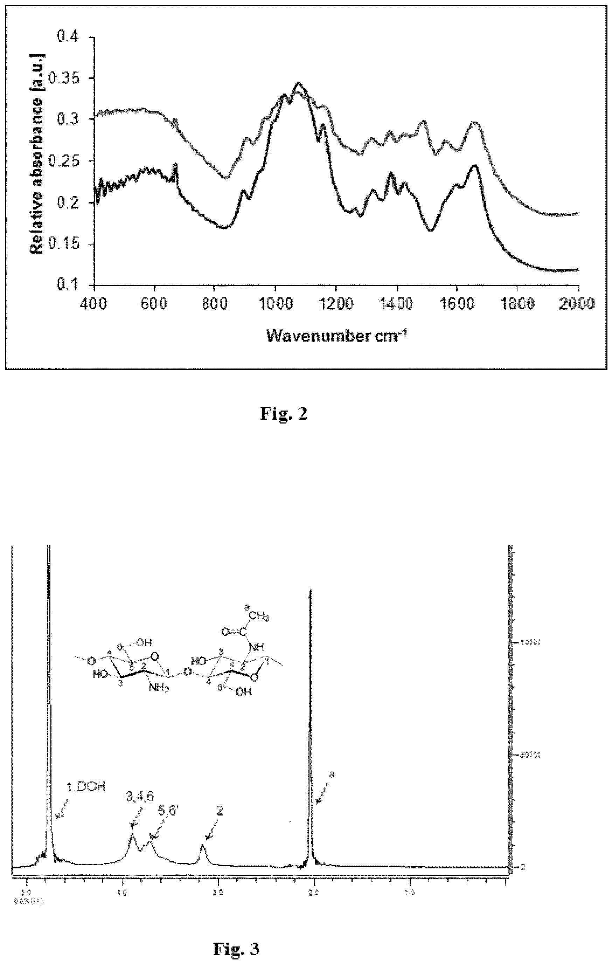

Modification of the polymer was carried out based on the method described previously in the literature (Kaminski K. et al. (2010). J Med Chem 53: 4141-4147). In the reaction of obtaining HTCC shown in Fig. 1, 2.5 g of chitosan was dispersed in 100 ml of distilled water then 10 ml of 0.5% acetic acid was added and the mixture was stirred for 30 min. In the next step 6.9 ml of glycidyltrimethylammonium *chloride* (GTMAC) was dropped in.

The reaction was carried out for 18 h at 55°C, while mixing with a magnetic stirrer. After this time, the reaction mixture was centrifuged at 4000 rpm for 10 minutes to remove suspended unmodified chitosan. Then, the supernatant was poured into the excess of acetone, and the resulting suspension was centrifuged at 4000 rpm for 20 min.

The supernatant was decanted, the precipitate was pre-dried in air and then dissolved in water. The resulting solution was centrifuged again, and the polymer which was dissolved in the supernatant was precipitated using a new batch of acetone. The dissolution and precipitation cycles were repeated two more times. The product HTCC obtained after the final precipitation was dried in a vacuum oven for 24 hours. The polymer with the degree of modification of 63%, defined as the percentage of glucose units substituted with GTMAC, was obtained.

The structure of cationically modified chitosan HTCC is confirmed by FTIR spectra shown in Fig. 2, where the darker line shows the spectrum of chitosan before modification, and the brighter line shows the spectrum of chitosan after modification (i.e. HTCC).

Within the FTIR spectrum of the modified chitosan two bands, among others, can be distinguished at 1520 cm-1, characteristic of the methyl groups,present in the modified chitosan, but not present in unmodified chitosan, and band at 1595 cm⁻¹, characteristic of the primary amines, observed in the spectrum of unmodified chitosan, but not present in the spectrum of the modified chitosan. In both cases, a band is observed at 1650 cm⁻¹, derived from the C = O in the primary amide bond present in the acetylated amino groups.

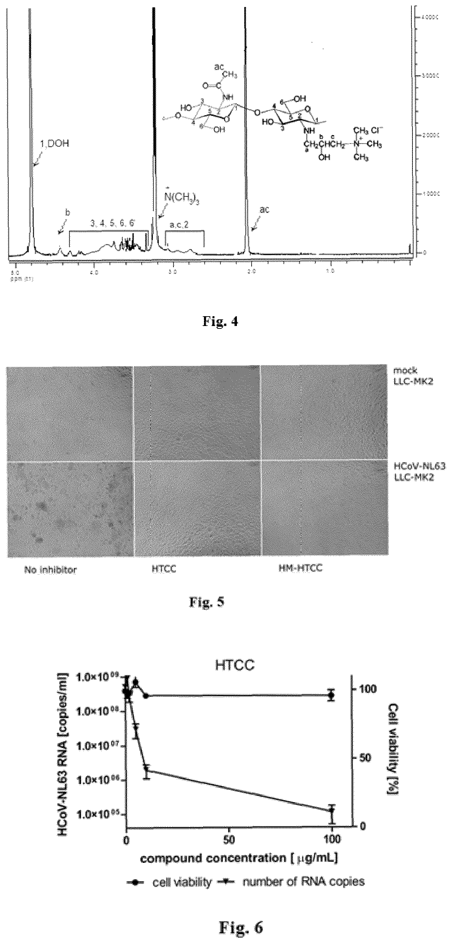

The structure of the modified chitosan (HTCC) was also confirmed with the use of 1 H-NMR spectra. 1H NMR measurements were performed in a mixture of D₂O and CD₃COOD. In the spectrum of the polymer after modification a band appeared at 3.2 ppm, characteristic of the protons of methyl groups linked to quaternary amine nitrogen. The 1H-NMR spectrum of the unmodified chitosan is shown in Fig. 3 and that of HTCC is shown in Fig.4.

### Example 2. Synthesis of the chitosan derivative HM-HTCC.

HTCC (1 g, 3 mmol of glucose units) was dissolved in a mixture of methanol and 1% acetic acid solution (pH = 5.5) in a 1:1 ratio. In 20 ml of methanol 0.3 mmol of N-dodecyl aldehyde and 1.257 g (20 mmol) of sodium cyanoborohydride (NaCNBH) were dissolved and added to the HTCC solution. The reaction mixture was stirred for 36 h at 20°C until a sol was formed. After the completion of the reaction, the polymer was precipitated using a 50:50 v/v mixture of methanol and diethyl ether. The precipitated polymer was washed with methanol and ether and dried in vacuum. The structure of the obtained product (HM - HTCC) was confirmed by measuring its 1H NMR spectrum.

### Example 3. Study on cytotoxicity of polymers HTCC and HM-HTCC and their anti-alphacoronaviral activity, on the example of human coronavirus NL63.

The cytotoxicity of polymers HTCC and HM-HTCC was studied using LLC-MK2 cells (ATCC CCL-7; cell line derived from epithelial cells of the Macaca mulatta kidney) sensitive to infection with the HCoV-NL63 virus and A549 cells (ATCC CCL-185 cell lines derived from lung cancer cells, Homo sapiens) (van der Hoek L. et al. (2004). Nat Med 10: 368-373; Schildgen O. et al. (2006). J Virol Methods 138: 207 - 210).

Cytotoxicity was determined using two assays. At first, a colorimetric test was carried on, based on the ability of the mitochondrial enzymes (succinate dehydrogenase) to reduce XTT dye (2,3-bis-(2-methoxy-4-nitro-5-sulfophenyl)-2H-tetrazolium-5-carboxanilide) to colored formazan salt.

Since there is a direct correlation between the cell viability and the amount of dye formed, by measuring the absorbance at the wavelength corresponding to the absorption maximum for this particular dye, it is possible to assess the condition of the cells. Evaluation of the cell viability was also carried out using neutral red dye.

Neutral red (NR) is passively transported into the cytoplasm of living cells and accumulates in lysosomes. Analysis of the number of viable cells and their condition is carried out by the colorimetric assessment of the sample, subsequently to cell lysis.

In the study LLC-MK2 or A549 cells were incubated for 6 days in minimal essential medium (MEM) with Hanks' and Earle's salts supplemented with 3% bovine serum, with increasing concentrations of HTCC or HM-HTCC polymer. After this time, the cytotoxicity was assessed using the two methods described above. In addition, cells were examined with a phase contrast microscope for the presence of a virus-specific alteration in the cell morphology. The results are shown in Table 1.

**Table 1. Results of cytotoxicity and cytopathicity test.**

| Cell Line | HTCC CC₅₀^{ab} | HTCC CC₅₀^{ac} | HM-HTCC CC₅₀^{ab} | HM-HTCC CC₅₀^{ac} |
|---|---|---|---|---|
| LLC-MK2 | 161.25 µg/ml | 191.92 µg/ml | 220.09 µg/ml | 215.23 µg/ml |
| A549 | 143.83 µg/ml | 156.46 µg/ml | 96.29 µg/ml | 96.13 µg/ml |

| | | | | |
|---|---|---|---|---|
| ^{a}CC₅₀ indicates the concentration of the polymer, which resulted in a decrease of cell viability by 50% ^{b}results obtained using the XTT test. ^{c}results obtained using the neutral red test. | | | | |

The results of the measurement conducted were consistent with observations of morphological changes in cells. Briefly, for both polymers CC₅₀ concentration was similar (∼200 µg/ml for LLC-MK2 cells and 100-150 µg/ml for A549 cells).

Inhibition of the human coronavirus NL63 replication by HTCC and HM-HTCC polymers was evaluated. The experiment was performed by the infection of sensitive cells (LLC - MK2) with HCoV-NL63 virus in the presence of polymers at increasing concentrations. Throughout experiment cells were cultured in MEM medium with Hank's and Earle's salts supplemented with 3% bovine serum.

Following the infection (two hours incubation of virus suspension on cells) the medium was replaced (MEM with Hanks' and Earle's salts and 3% bovine serum) containing HTCC or HM-HTCC polymer at a specific concentration. Assessment of morphological changes was carried out using a phase contrast microscope 6 days post-infection. In the presence of HTCC polymer (10 µg/ml) inhibition of viral replication was observed and no cytopathic effect related to the virus replication was noted. Similarly, in the presence of HM-HTCC polymer (50 µg/ml) the inhibition of viral replication and no cytopathic effect were observed (Fig. 5).

On the contrary, the analysis carried on using unmodified chitosan revealed no inhibition of HCoV-NL63 replication by this polymer. Similarly, cationic derivatives of other polymers (e.g. dextran or PAH) showed no visible, anticoronaviral inhibitory activity.

The effect of HTCC and HM - HTCC polymers on the replication of HCoV - NL63 was also analyzed using the real-time, quantitative RT-PCR. For this purpose the quantitative method described previously was used (Pyrc K.et al. (2006). Antimicrob Agents Chemother 50: 2000-2008). In the study LLC-MK2 cells were incubated for 6 days in MEM medium with Hank's and Earle's salts, supplemented with 3% bovine serum. Under infection, after 2 hours of incubation of the cells with the virus suspension, medium was removed, cells were washed with sterile phosphate buffered saline and overlayed with fresh medium (MEM with Hanks' and Earle's salts, 3% bovine serum) containing HTCC or HM-HTCC polymer at the specific concentration. After the incubation, the total RNA was isolated from the cell supernatants and following the reverse transcription reaction used as template for quantitative PCR.

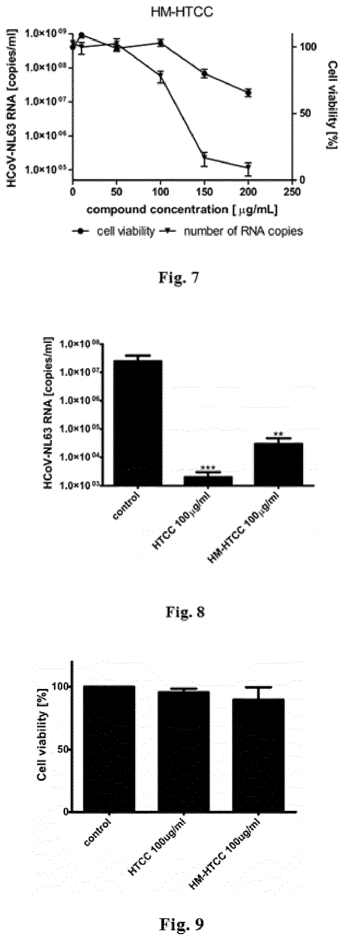

The experiment was carried out for increasing concentrations of the polymers. The results are shown in Fig. 6 and 7 and in Table 2. It was observed that HTCC and HM-HTCC polymers caused the inhibition of HCoV-NL63 replication, while the virus replicated in the reference sample. Analysis of the datae on cytotoxicity (CC₅₀) and antiviral activity (IC₅₀) enabled estimation of selectivity indexes, which are also included in Table 2.

Graphs illustrating the concentration-dependent cytotoxicity and inhibition of virus replication by the HTCC polymer or by the HM-HTCC polymer are shown in Fig. 6 and Fig. 7, respectively.

**Table 2. Inhibition of viral replication of HCoV-NL63 virus by HTCC and HM-HTCC polymers.**

| Polymer | IC₅₀ | Selectivity index SI₅₀ |
|---|---|---|
| HTCC | 2.75 ± 1.18 _{µ}g/ml [13.41 nM] | 58.64 |
| HM-HTCC | 68.52 ± 18.71 µg/ml [308.65 nM] | 3.21 |

As clearly visible, the HTCC polymer shows activity at significantly lower concentrations, which results in better selectivity index. On the structural level, HTCC and HM-HTCC are similar, except for the hydrophobic groups present exclusively in the HM-HTCC polymer. It is therefore possible, that the attachment of these groups limits accessibility of the HTCC core or alters its properties.

### Example 4. Study on cytotoxicity of polymers HTCC and HM-HTCC and their anti-alphacoronaviral activity, on the example of human coronavirus NL63 utilizing fully differentiated human airway epithelial (HAE) cultures.

To determine whether the observed anti- alphacoronaviral effect is cell-specific, the inhibitory activity of the polymers in a more natural environment was assessed; HAE cultures, mimicking natural conductive airway epithelium, were used for this purpose. HAE cultures are formed by the multi-layered, fully differentiated primary human airway epithelial cells growing on the collagen-coated plastic supports on air/liquid interphase. Briefly, human tracheobronchial epithelial cells were expanded on plastic to generate passage 1 cells and plated at density of 3 × 10⁵ cells per well on permeable Transwell inserts (6.5-mm-diameter) supports. Human airway epithelium (HAE) cultures were generated by provision of an air-liquid interface for 6 to 8 weeks to form well-differentiated, polarized cultures that resemble *in vivo* pseudostratified mucociliary epithelium.

HAE cultures were infected with HCoV-NL63 by inoculation of the infectious material onto the apical surface. Following 2 h incubation at 32 °C, the unbound virus was removed by 1 × PBS washing (two times, 100 µl each) and HAE cultures were maintained at an air-liquid interface for the rest of the experiment. Further, the apical surfaces of HCoV-NL63-infected and mock-infected HAE cultures were rinsed every 24 h with 100 µl of the medium containing a given inhibitor.

Cytotoxicity was determined using a colorimetric test based on the ability of the mitochondrial enzymes (succinate dehydrogenase) to reduce XTT dye (2,3-bis-(2-methoxy-4-nitro-5-sulfophenyl)-2H-tetrazolium-5-carboxanilide) to colored formazan salts.

To analyze replication of HCoV-NL63, 72 h post infection, 100 µl of 1xPBS was applied to the apical surface of HAE and collected following 10 min. incubation at 32°C.

Graphs illustrating the inhibition of virus replication by the HTCC an HM-HTCC polymers and the cytotoxicity of polymers on HAE cultures (as determined by the XTT assay) are shown in Fig.8 and 9 respectively.

### Example 5. Study on cytotoxicity of polymers HTCC and HM-HTCC and their antibetacoronaviral activity, on the example of murine hepatitis virus (MHV).

Cytotoxicity of HTCC and HM - HTCC polymers was analyzed on LR7 cells susceptible to MHV virus infection (Kuo L.et al. (2000). J Virol 74: 1393-1406; Rossen J.W. et al. (1996). Virology 224: 345-351). Cytotoxicity was determined using a colorimetric test based on the ability of the mitochondrial enzymes (succinate dehydrogenase) to reduce XTT dye (2,3-bis-(2-methoxy-4-nitro-5-sulfophenyl)-2H-tetrazolium-5-carboxanilide) to colored formazan salts.

Since there is a direct correlation between the cell viability and the amount of dye formed by measuring the absorbance at the wavelength corresponding to the absorption maximum for this particular dye, it is possible to assess the condition of the cells. Evaluation of the cell viability was also carried out using neutral red dye.

Within the study, LR7 cells were incubated for 48 hours in the defined Dulbecco's Modified Eagle Medium (DMEM) supplemented with 3% bovine serum, with increasing concentrations of HTCC or HM-HTCC polymer. After this time, the cytotoxicity was assessed in accordance with the above description. In addition, cells were inspected for morphological alterations using a phase contrast microscope.

The obtained results are shown in Table 3.

**Table 3. Cytotoxicity of HTCC and HM-HTCC polymers**

| Cell line | HTCC CC₅₀^{ab} | HM-HTCC CC₅₀^{ac} |
|---|---|---|
| LR-7 | > 100 µg/ml | > 100 µg/ml |

| | | |
|---|---|---|
| ^{a}CC₅₀ indicates the concentration of the polymer, which resulted in a decrease of cell viability by 50% ^{b}results obtained using the XTT test ^{c}results obtained using the neutral red test | | |

The inhibition of coronavirus MHV replication was analyzed with the use of HTCC and HM-HTCC polymers. The experiment was performed by infecting sensitive cells (LR7) with MHV virus in the presence of the polymers. For the whole duration of the assay, cells were maintained in the DMEM medium supplemented with 3% bovine serum. After two hours of cell incubation with the virus, the medium was removed, cells were washed with sterile phosphate buffered saline and overlayed with fresh medium (DMEM with 3% bovine serum) containing the HTCC or HM-HTCC polymer.

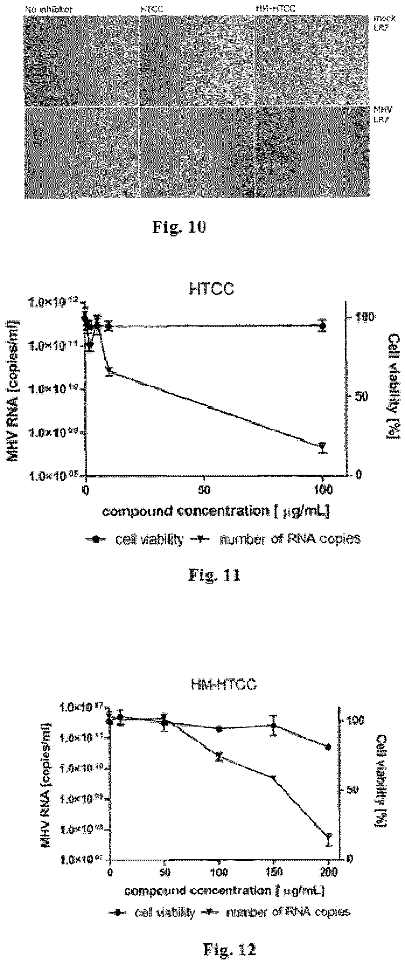

Cells were visually inspected for morphological alterations using a phase contrast microscope, 48 hours post-infection (Fig.10). The impact of HTCC and HM-HTCC polymers on the replication of MHV virus was also analyzed using the RNA copy number calculation in the medium via RT-PCR analysis in real time. The quantitative method was applied with the use of 5'MHV_NF TGG CCG AAG AAA TTG CTG CTC TTG, 3' MHV_NR GCC TGA CTT CTT TGG TGG CAC TTT GCT primers and FAM / TAMRA MHV_Np TTT GGC TAA GCT CGG TAA AGA TGC CG fluorescent probe.

The reaction was carried out under the same conditions as previously described (Pyrc K. et al. (2006). Antimicrob Agents Chemother 50: 2000-2008).

The experiment was carried out for increasing concentrations of the polymers. The results are shown in Fig. 11 and 12 and in Table 3. It was observed that HTCC and HM-HTCC polymers caused the inhibition of MHV virus replication, while the virus replicated in the reference sample.

Graphs illustrating the concentration-dependent cytotoxicity and inhibition of virus replication by the HTCC polymer or by the HM-HTCC polymer are shown in Fig. 11 and Fig. 12, respectively.

### Example 6. Interaction between HTCC polymer and the spike protein of the HCoV-NL63 coronavirus (Se-NL63).

To study the interaction between Se-NL63 and HTCC, the polymer was labeled with fluorescein according to the following procedure. 150 mg of HTCC was dissolved in a mixture of 5.0 ml of distilled water and 15.0 ml of DMSO. Five milligrams of fluorescein isothiocyanate (FITC) was dissolved in 1 ml of acetone, the solution was added to the sample containing HTCC, and vigorously mixed for 24 h in the dark. Subsequently, the labeled polymer (FITC-HTCC) was precipitated with acetone and isolated by centrifugation for 5 min at 4000 rpm. The precipitate was washed with acetone, dissolved in water, dialyzed against water for 2 days and further against acetone:water mixture (1:4 v/v) for 1 day and freeze dried. The degree of substitution of FITC-HTCC , defined as the number of FITC groups per a glucose group of HTCC, was found to be 1.66% based on the UV-VIS spectra.

The fluorescence spectra of FITC-HTCC (at c_{FITC-HTCC} = 0.5 g/ml) in water were assessed in a control sample and in samples containing increasing concentrations of Se-NL63 or bovine serum albumin (BSA). The measurement was conducted using λₑₓ = 494 nm. It was verified that Se-NL63 protein and other components of the sample do not absorb at this excitation wavelength and do not interfere with the FITC-HTCC fluorescence intensity.

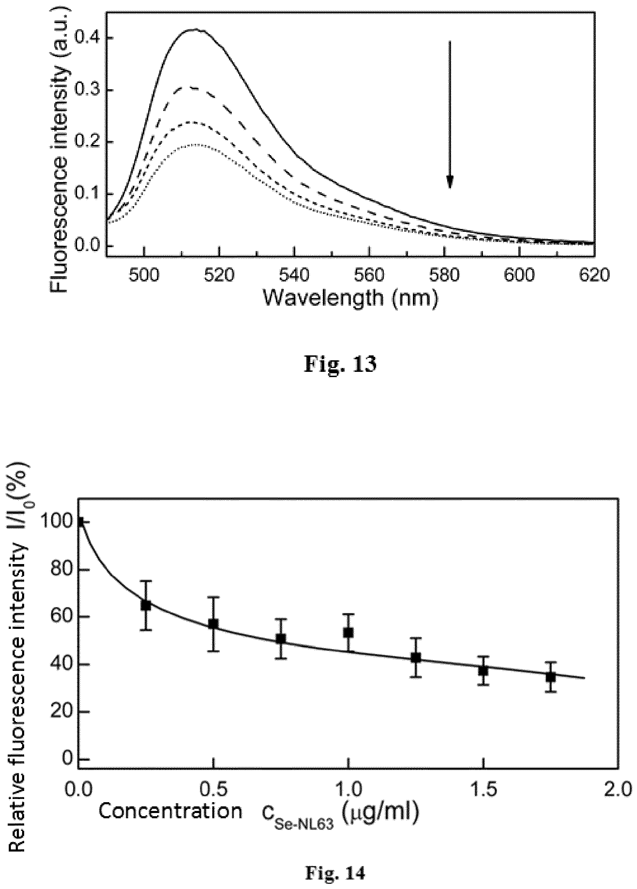

The fluorescence intensity of FITC-HTCC decreased significantly with growing concentration of Se-NL63 (Fig. 13 and 14). Strong quenching of FITC-HTCC fluorescence by Se-NL63 indicates a strong interaction (complex formation or binding) between Se-NL63 and FITC-HTCC. No decrease in the fluorescence intensity of FITC-HTCC was observed in the presence of bovine serum albumin (BSA) indicating that BSA does not interact with FITC-HTCC and, consequently, that there is some specificity in the interaction between Se-NL63 and FITC-HTCC.

Graphs illustrating fluorescence spectra of FITC-HTCC in the presence of 0, 1.0, 2.0, and 2.5 g/ml of Se-NL63 and the dependence of the relative fluorescence intensity (I/I₀) of FITC-HTCC at ∼λ = 513 nm on Se-NL63 concentration (λ∼ₑₓ = 494 nm, c_{FITC-HTCC} = 0.5 g/ml) are shown in Fig.13 and Fig. 14 respectively.

## Claims

1. Chitosan polymers HTCC and HM-TCC of the formula I 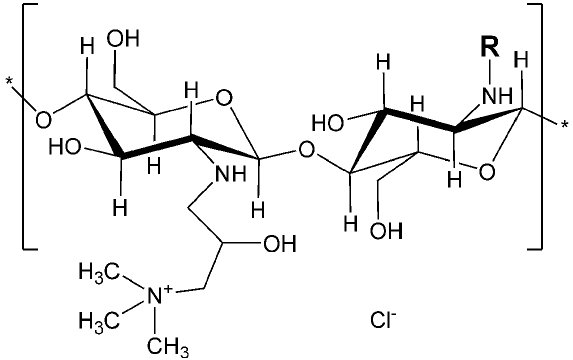 wherein R is selected from H, COCH₃ (HTCC) or from H, COCH₃, C12H25 (HM-HTCC) for use in the treatment and prevention of infections caused by coronaviruses.

2. The chitosan polymers for use according to claim 1 in the treatment and prevention of infections caused by alphacoronaviruses.

3. The chitosan polymers for use according to claim 2 in the treatment and prevention of infections caused by HCoV-NL63.

4. The chitosan polymers for use according to claim 1 in the treatment and prevention of infections caused by betacoronaviruses.

5. The chitosan polymers for use according to claim 4 in the treatment and prevention of infections caused by murine coronavirus MHV.

6. The chitosan polymers for use according to claims 1 to 5 in the treatment and prevention of respiratory infections manifested as insufficiency and impairment of respiratory functions in humans or animals.

7. The chitosan polymers for use according to claim 6 in the treatment and prevention of infections selected from the group:
- upper respiratory tract infections;
- lower respiratory tract infections;
- croup in children;
- gastrointestinal tract infections;
- nervous system infections;
- Kawasaki disease.

8. The chitosan polymers for use according to claim 1, **characterized in that** the chitosan polymer HTCC is obtained by reacting chitosan with glycidyltrimethylammonium chloride.

9. The chitosan polymers for use according to claim 1, **characterized in that** the chitosan polymer HTCC is further modified by substituting by hydrophobic groups yielding HM-HTCC polymer.

10. The chitosan polymers for use according to claim 9, **characterized in that** the hydrophobic groups are n-dodecyl groups.

11. The chitosan polymers for use according to claim 1, **characterized in that** they are used as a solution in the form of a liquid or an aerosol administered to the respiratory tract.

## Patentansprüche

1. Chitosanpolymere HTCC und HM-TCC mit der Formel I 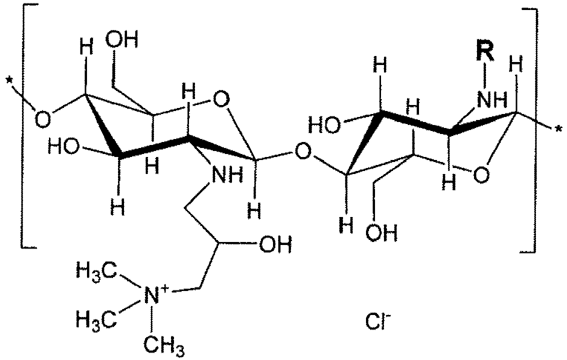 wobei R aus H, COCH₃ (HTCC) oder aus H, COCH₃, C₁₂H₂₅ (HM-HTCC) ausgewählt ist, zur Benutzung zur Behandlung von und Vorbeugung gegen Infektionen, die durch Coronaviren verursacht werden.

2. Chitosanpolymere zur Benutzung nach Anspruch 1 zur Behandlung von und Vorbeugung gegen Infektionen, die durch Alphacoronaviren verursacht werden.

3. Chitosanpolymere zur Benutzung nach Anspruch 2 zur Behandlung von und Vorbeugung gegen Infektionen, die durch HCoV-NL63 verursacht werden.

4. Chitosanpolymere zur Benutzung nach Anspruch 1 zur Behandlung von und Vorbeugung gegen Infektionen, die durch Betacoronaviren verursacht werden.

5. Chitosanpolymere zur Benutzung nach Anspruch 4 zur Behandlung von und Vorbeugung gegen Infektionen, die durch murines Coronavirus MHV verursacht werden.

6. Chitosanpolymere zur Benutzung nach Anspruch 1 bis 5 zur Behandlung von und Vorbeugung gegen Atemwegsinfektionen, die sich als Insuffizienz und Beeinträchtigung von Atemfunktionen bei Menschen oder Tieren manifestieren.

7. Chitosanpolymere zur Benutzung nach Anspruch 6 zur Behandlung von und Vorbeugung gegen Infektionen, ausgewählt aus der Gruppe:
- Infektionen der oberen Atemwege;
- Infektionen der unteren Atemwege;
- Krupp bei Kindern;
- Infektionen des Magen-Darm-Traktes;
- Infektionen des Nervensystems;
- Kawasaki-Krankheit.

8. Chitosanpolymere zur Benutzung nach Anspruch 1, **dadurch gekennzeichnet, dass** das Chitosanpolymer HTCC durch Zur-Reaktion-Bringen von Chitosan mit Glycidyltrimethylammonium gewonnen ist.

9. Chitosanpolymere zur Benutzung nach Anspruch 1, **dadurch gekennzeichnet, dass** das Chitosanpolymer HTCC ferner durch Substituieren mit hydrophoben Gruppen modifiziert ist, was HM-HTCC-Polymer ergibt.

10. Chitosanpolymere zur Benutzung nach Anspruch 9, **dadurch gekennzeichnet, dass** die hydrophoben Gruppen n-Dodecylgruppen sind.

11. Chitosanpolymere zur Benutzung nach Anspruch 1, **dadurch gekennzeichnet, dass** sie als eine Lösung in Form einer Flüssigkeit oder eines Aerosols benutzt werden, die/das an die Atemwege verabreicht wird.

## Revendications

1. Polymères de chitosan HTCC et HM-TCC de la formule I : 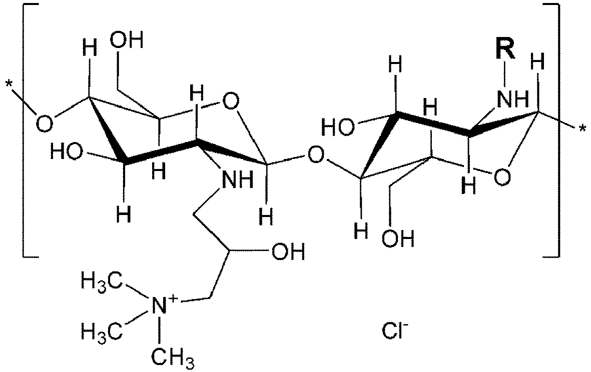 dans laquelle R est choisi parmi H, COCH₃ (HTCC) ou parmi H, COCH₃, C₁₂H₂₅ (HM-HTCC) pour une utilisation dans le traitement et la prévention d'infections provoquées par les coronavirus.

2. Polymères de chitosan pour une utilisation selon la revendication 1 dans le traitement et la prévention d'infections provoquées par les alphacoronavirus.

3. Polymères de chitosan pour une utilisation selon la revendication 2 dans le traitement et la prévention d'infections provoquées par HCoV-NL63.

4. Polymères de chitosan pour une utilisation selon la revendication 1 dans le traitement et la prévention d'infections provoquées par les bêtacoronavirus.

5. Polymères de chitosan pour une utilisation selon la revendication 4 dans le traitement et la prévention d'infections provoquées par le coronavirus murin MHV.

6. Polymères de chitosan pour une utilisation selon l'une des revendications 1 à 5 dans le traitement et la prévention d'infections respiratoires se manifestant comme une insuffisance et une détérioration de fonctions respiratoires chez les êtres humains ou les animaux.

7. Polymères de chitosan pour une utilisation selon la revendication 6 dans le traitement et la prévention d'infections choisies dans le groupe :
- infections du tractus respiratoire supérieur ;
- infections du tractus respiratoire inférieur ;
- croup chez les enfants ;
- infections du tractus gastro-intestinal ;
- infections du système nerveux ;
- maladie de Kawasaki.

8. Polymères de chitosan pour une utilisation selon la revendication 1, **caractérisés par le fait que** le polymère de chitosan HTCC est obtenu par réaction de chitosan avec le chlorure de glycidyltriméthylammonium.

9. Polymères de chitosan pour une utilisation selon la revendication 1, **caractérisés par le fait que** le polymère de chitosan HTCC est encore modifié par substitution par des groupes hydrophobes fournissant le polymère HM-HTCC.

10. Polymères de chitosan pour une utilisation selon la revendication 9, **caractérisés par le fait que** les groupes hydrophobes sont des groupes n-dodécyle.

11. Polymères de chitosan pour une utilisation selon la revendication 1, **caractérisés par le fait qu'**ils sont utilisés en tant que solution sous la forme d'un liquide ou d'un aérosol administré au tractus respiratoire.
